# EUROPEAN PATENT APPLICATION

(11) **EP 4 024 406 A1**
(43) Date of publication of application: **06.07.2022**
(21) Application number: 20217953.7
(22) Date of filing: 31.12.2020
(51) Int. Cl.: G16H 50/20, G16H 50/30, G16H 50/50

(54) **ANALYTICAL PLATFORM FOR THE PROVISION OF SOFTWARE SERVICES ON THE CLOUD**

(30) Priority: 30.12.2020 IT 202000032753
(71) Applicant: Kazaam Lab s.r.l., 90142 Palermo (IT)
(72) Inventor: Morfea, Salvatore, 90142 Palermo (IT); Panni, Simona, 00164 Roma (IT); Rombo, Simona Ester, 90142 Palermo (IT)
(74) Representative: De Tullio, Michele Elio

(57) **Abstract**

The present invention describes an analytical platform based on a microservices architecture and a method based on it, to provide software services in a cloud environment for the integration, modeling and analysis of data. The platform is structured so as to be divided into a series of microservices, which through the implementation of specific algorithms, perform the integration of a plurality of data types from a plurality of heterogeneous sources, the modeling of said plurality of types of data through the creation of specific functional networks, the processing of said plurality of data types for the extraction of hidden information, the generation of suggestions and predictions based on the generated knowledge.

The platform is organized according to a "multi-sided" business model, which involves three different categories of users, independent of each other: doctors and analysts who work in clinical institutes in the field of Precision Medicine, clinical laboratories, pharmaceutical companies. The platform integrates heterogeneous biological data, such as molecular, genetic, clinical and phenotypic data, information on stem cells, and many others, integrating them from various sources, such as public and private databases, in order to assist the activity of doctors and analysts in the choice of customized therapies, in the discovery or reallocation of new drugs, in the choice of clinical analyses best suited to the types of patients.

## Description

### TECHNICAL FIELD

The present invention refers to integrated systems for the provision of software services on the cloud. Particularly, the present invention proposes an analytical platform, which provides software services in a cloud environment for the integration, modeling and analysis of data, in order to support the work of doctors and other health professionals. The platform based on Big Data technologies, Artificial Intelligence and Real Time Analytics integrates heterogeneous, difficult to interpret data from a variety of sources in order to extract hidden information regarding the effect of gene mutations on phenotype and on the probability of disease occurrence that may be useful in the interpretation and resolution of the most complex clinical cases.

### BACKGROUND

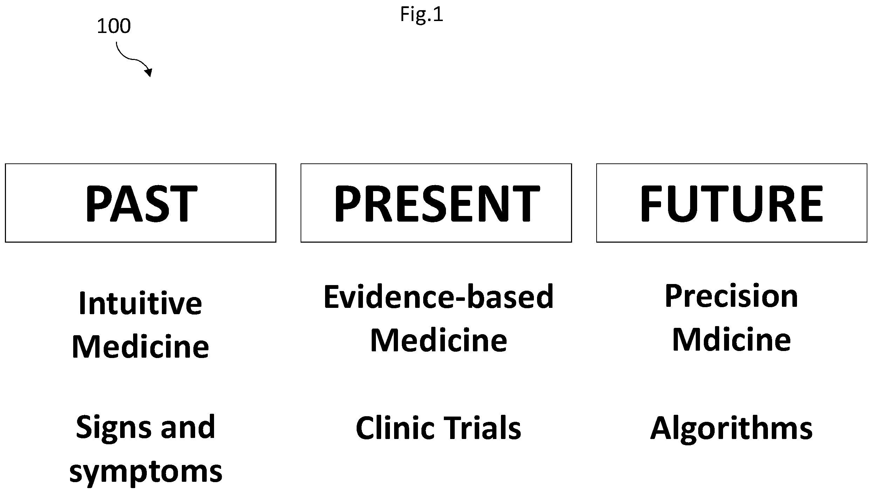

Since the 1990s, medicine has been based on so-called evidence of effectiveness (Evidence-Based Medicine), namely the integration of the doctor's clinical experience with the patient's informed opinion and research data. In a way, the doctor's decision-making ability was conditioned by the use of these 3 factors. Nowadays, this type of medicine is gradually being replaced by value-based healthcare (Value-Based Healthcare), based on the principles of maximizing benefits through personalized treatment based on Precision Medicine (Figure 1). Precision Medicine (MP) develops a more personalized approach to both prevention and patient diagnosis and treatment. Based on the concept that each person is different, this medicine also examines how each patient reacts to his or her disease and the treatment administered through the drugs. The resulting benefit is received primarily by the patient, who gains better adherence to treatment, but it also impacts on the entire healthcare system. In order to make this possible, it is necessary to analyze a range of information from different sources about the patient, the diseases and the drugs. This information can be found in both public and private databases and forms the basis for more specialized services aimed at different market segments. This refers, for example, to data concerning cellular interactions, or genotype-phenotype associations, or exome sequencing. Only in this way is it possible to identify the medical treatment that is best suited to each specific pathology and to correct it quickly, adapting it as precisely as possible to the reactions and behavior of each individual patient. But precisely because it is necessary to bring together very different information, covering aspects of cell life, diseases and phenotypes, the corresponding data are very heterogeneous and require very specific skills to be processed and analyzed correctly. For this reason, MP has not yet found the right diffusion and application in both medical and pharmacological fields, although important pharmaceutical companies and technological industries are promoting progress in this direction. We can summarize the benefits of MP as follows:
- greater accuracy in diagnosis
- better treatment results
- greater tolerance to drugs
- better knowledge of risk factors
- better management of side effects.

Having a system that allows the application of personalized medicine solutions, integrating data from individual users with data from public databases maintained by scientists around the world, would make it possible:
- access to accurate data on the direct and indirect effects of drugs on patients,
- access to increased knowledge to implement forms of preventive medicine,
- access to increased knowledge to implement forms of predictive medicine,
- the availability of more precise data and information for the benefit of clinical studies. Recent years have seen the proliferation of digital solutions based on Artificial Intelligence, applied in many fields of technology, which are able to accelerate the process of data verification and control, as required in medical and pharmacological applications. These solutions, based for instance on 'deep learning' methodologies, which were already in use in many areas of medicine, including radiology, pathology, genomic medicine, cardiology, outpatient services and intensive care, are now widely used in the automated interpretation of biomedical images.

For example, international patent application WO2020227794 describes a system and method for analyzing mammographic images. In particular, the application provides a device to classify the quality of breast position and breast density in the mammographic image, ranking the risk of breast density preventing the diagnosis of a possible lesion. "Deep learning" methodologies are based, for example, on Artificial Neural Networks (ANNs), namely hardware and/or software systems whose functioning is inspired by the neural networks of the human brain. ANNs train through training data, which are fed to machine learning algorithms ("machine learning"). The greater the number of learning data analyzed, the better will be the process of self-perfection of the algorithm.

The creation of 'Expert Systems', namely systems based on the use of Artificial Intelligence (AI) technologies and methods, developed specifically for the medical field and thus for digital health, has been developing since the 1970s. MYCIN, for example, was one of the first expert systems, or artificial intelligence programs, to be used for the treatment of blood infections. In 1972, work began on MYCIN at Stanford University in California, to diagnose patients on the basis of reported symptoms and medical test results. Unlike ANNs, expert systems use a knowledge base, structured according to rules, namely they use an inferential engine to apply precise logical rules to this knowledge base in order to extrapolate further knowledge. One of the disadvantages of the use of the ANNs resided, in fact, in the lack of these predetermined rules and in their behavior as a "black box", that is, a black box that, starting from input data supplies output results, with little possibility to verify the modalities with which a certain result has been reached and the truthfulness of the conclusions. Expert systems, with their ability to move from an initial condition based on the patient's simple condition to the formulation of a precise final diagnosis, with the recognition of a given disease, are the best candidates for application to new MP. For this reason, much research effort has been spent on the development of these systems, but very few have reached routine medical use. For example, the Watson platform, a system designed within the IBM group, is focused on creating an artificial intelligence capable of handling large amounts of unstructured data. Watson has been applied, for example, in some pilot studies for the discovery of new targets and/or the reallocation of drugs. Many of the systems developed so far mainly provide services for diagnostics based on integration and analysis of digital text and images, or decision support using only sequence data and in any case in the context of very specific diseases (e.g. tumors of various kinds).

For example, US patent US10332639 (B2) provides a network system and a method for using it to exchange information between users simultaneously, sharing a variety of multimedia data, including medical images, streaming video, audio, drawings and annotations, in both live and archived modes.

An important prerogative of these systems concerns the use of the 'Cloud', namely personal storage spaces on the Internet, for the distribution of computing services, such as servers, or as storage and database resources. It has become vital to offer flexible resources and economies of scale, which are essential for analyzing large data sets using Artificial Intelligence methods in biomedicine and healthcare.

For example, patent KR102179586 (B1) relates to a "cloud platform" system for reading medical images, comprising a plurality of pre-programmed and modularized image processing modules for performing pre-processing of medical images, a plurality of pre-programmed and modularized artificial intelligence modules with artificial intelligence algorithms, a storage unit in which a plurality of data sets classified according to body part, type of disease, type of image size is stored.

For example, another important application of these systems concerns genomic diagnostics, where the search for genotype-phenotype interrelationships between syndromes, especially for extremely rare diseases, requires a great deal of work on the part of doctors and geneticists.

Another field of application concerns healthcare systems, which produce huge amounts of information, such as patient demographic and clinical data, and need new Artificial Intelligence based techniques to extract new knowledge from them. See for example the article: *'AI based healthcare platform for real time, predictive and prescriptive analytics using reactive programming,* J Kaur, KS Mann, Journal of Physics: Conference Series, 2017'. The article shows the importance of applying AI based predictive and prescriptive analytics techniques in healthcare. In this regard, it should be noted that all existing applications that integrate data and apply Artificial Intelligence for their analysis refer exclusively to data from medical reports, medical records and biological sequences. On the other hand, many of the problems that need to be addressed in order to put Precision Medicine into practice involve huge amounts of 'omics' data, namely from the 'omics' sciences (interactomics, proteomics, genomics, transcriptomics, epigenomics, etc.) and the way in which such omics data can be related to each other. Such 'Big Data' in the medical and biological domain provide unprecedented opportunities to work on interesting problems, but also raise many new issues related to the extraction of useful knowledge, its organization and analysis, the security of information and the usefulness of existing techniques when moving into the Big Data domain (V. Marx. Biology: The big challenges of big data. Nature, 498(7453):255-260, 2013). Some of the typical problems in the domain of interest, especially when data become large, relate to the fact that cellular components often cannot be analyzed independently of the relationships between them (D. von Mering et al. Comparative assessment of a large-scale data sets of protein-protein interactions. Nature, 417(6887):399-403, 2002). This is true both in the case of components (e.g. proteins) that contribute to the performance of a specific biological function, and in the case of components (e.g. genes) whose mutations play a precise role in the emergence of a disease. Appropriate models are therefore needed to represent them, and techniques suitable for managing complex and heterogeneous data are needed to analyze them. In addition, there are many potential applications of systems for analyzing medical and biological Big Data in the pharmaceutical field. A fully integrated system, therefore, which allows the integration, modelling and analysis of data from various sources, including omics data, phenotypic data, behavioral data, stem cell data, "drug target" associations, and which can be used by various distinct categories of users, such as, for example, physicians and analysts working within clinical institutes, clinical laboratories for the analysis of clinical results, pharmaceutical companies for automatic support in "drug design" and reduction of drug-related "side effects", is strongly required.

### SUMMARY

The present invention describes an analytical platform based on a microservices architecture configured to provide a plurality of users in a cloud environment the availability of software services for integration, modelling and analysis of data, with the aim of extracting useful information to identify the best treatment and to support the work of doctors and other health professionals.

According to an embodiment of the invention, the analytical platform, based on a microservices architecture, configured to provide the availability of software services in a cloud environment, is based on the combination of Artificial Intelligence, Big Data and Real-time Analytics techniques and integrates data from a plurality of heterogeneous sources, such as public and private databases and independent collection sources, said data comprising omics data, clinical data, behavioural data, "drug-target" associations and phenotypic data.

According to one embodiment of the invention, the analytical platform is configured to provide a set of algorithms implemented as microservices to generate special functional network models, said networks being capable of simultaneously linking a plurality of heterogeneous information, said information comprising molecular interactions, gene expression, transcriptomics, stem cells, epigenomics, clinical data and phenotypic data, and "drug-target" associations.

According to an embodiment, the analytical platform, configured to provide a plurality of users in a cloud environment with the availability of software services for data integration, modelling and analysis, comprises the following modules for implementing the various data processing steps: a user interface through which the user enters requests; an API (Application Program Interface) interface program comprising at least one microservice for authorising the individual user and directing requests from said individual user to one or more microservices; a main module, or "Application Core" of the system, comprising one or more microservices, for integrating a plurality of heterogeneous biological data from different sources, linking together the various interactions between said plurality of data, facilitating the creation by said individual user of specific functional networks for performing custom analyses.

According to a particular form of implementation, the main module comprises three essential microservices: the "Scheduler microservice", through which the "download" of data takes place, the "Parser microservice" for processing the data, and the "Graph microservice" for creating the integrated functional network.

The present invention relates to a method for providing availability of software services in a cloud environment comprising integrated use of a plurality of data types, construction of complex functional networks, comprising information from molecular interactions, gene expression, transcriptomics, stem cells, epigenomics, clinical data and phenotypic data, and application of Artificial Intelligence algorithms, for providing support to physicians in the field of Precision Medicine, said method comprising: providing a Big Data Analytics platform based on a microservices architecture for implementing the data integration, modelling and analysis steps in a cloud environment, wherein said data integration steps comprise having a user interface for request entry by an individual user, receiving and managing requests from the user interface via an API Gateway interface program, directing said requests to one or more connected microservices; said modelling and analysis steps include the integration of a main module, or "Application Core" of the system to link together the various information coming from said plurality of data sources, facilitate the creation of specific functional networks integrating the totality of input information for the realisation of customised analyses.

According to an embodiment of the present method, said plurality of data types integrated through the platform is modelled through complex functional networks and on the basis of this integration and modelling of the data, the platform performs complex analyses that enable the provision of various services to support the activities of doctors and other healthcare professionals, said services comprising the calculation of risk factors associated with the occurrence of diseases and possible "side effects", the suggestion of specific clinical analyses to be required depending on the situation, the discovery of new targets and/or the reallocation of drugs.

According to a further embodiment of the method, said plurality of data types concerns cellular interactions (e.g., protein-protein, mRNA-protein, etc.), biological functionalities (e.g., derived from biological ontologies), genotype-phenotype associations (e.g., gene-disease), sequencing (e.g., RNAseq).

The aim of the present invention is to support on a large scale the activity of doctors and healthcare professionals in the field of Precision Medicine, to enable them to formulate personalised therapies, even in those cases where this is not possible due to lack of sufficient information, such as in cases of not fully defined protocols, patients with different characteristics and phenotypes, lack of patient data.

The aim of the present invention is also to address different categories of users, such as:
- doctors and analysts working within clinical institutes in the field of Precision Medicine,
- clinical laboratories,
- pharmaceutical companies.

The advantages offered by the present invention comprise:
- the combination of patient data, including those that are difficult to categorise, with aggregated data from public databases, a combination that makes it possible to extrapolate useful information to identify the best therapies to follow - no other similar tools capable of performing the same combination have emerged from the literature
- the integration and modelling of molecular data, not exclusively belonging to sequence data, in order to perform customised analyses at the user's request;
- the inclusion of specific functionalities for integration, modelling and analysis of stem cell data - these are specialised services for automatic processing of stem cell data, which distinguish the present invention from those existing in the state of the art;
- integration of patient behavioural data with molecular data, to achieve more effective categorisation of patients - no other similar tools capable of integrating behavioural information with phenotypic and genotypic data have been found in the literature.

So far, the known systems were configured to provide diagnostic services based on the integration and analysis of digital texts and images, or decision support for doctors and analysts based only on sequence data, and in any case always in the context of very specific diseases (e.g. cancers of various kinds). The present invention makes it possible to broaden the support to doctors working in Precision Medicine, allowing the integration of a much wider range of data types, and adapting them to even the most complex cases, thus enabling the identification of personalised therapies even in those cases where today's technologies do not provide useful solutions. This is possible thanks to the adoption of functional network models, peculiar to the platform, which make it possible to link together information that might otherwise be incorrectly unrelated.

For example, different diseases often have different genetic markers associated with them, so direct interrogation of the sources where marker and disease data are contained would lead to the conclusion that diseases with different markers have nothing in common. For some of them, however, the markers, although different, are linked through 'short pathways' within the complex functional networks built by the platform, which denotes that these diseases may instead be related, as confirmed by experimental validation carried out on the system. Important consequences of this include the fact that one disease may become a "side effect" of another depending on the drug therapy prescribed. In order to properly integrate the data by modelling them as functional networks, since the resulting networks are of enormous size, interaction with "Big Data" technologies and hardware (cloud) in the distributed environment is crucial.

For the reasons discussed above, the claimed product is highly innovative and competitive in the Precision Medicine market.

### SHORT DESCRIPTION OF THE DRAWINGS

Fig.1 shows schematically the evolution of medicine over time, according to three evolutionary stages (100): Intuitive Medicine, Evidence-based Medicine and Precision Medicine.

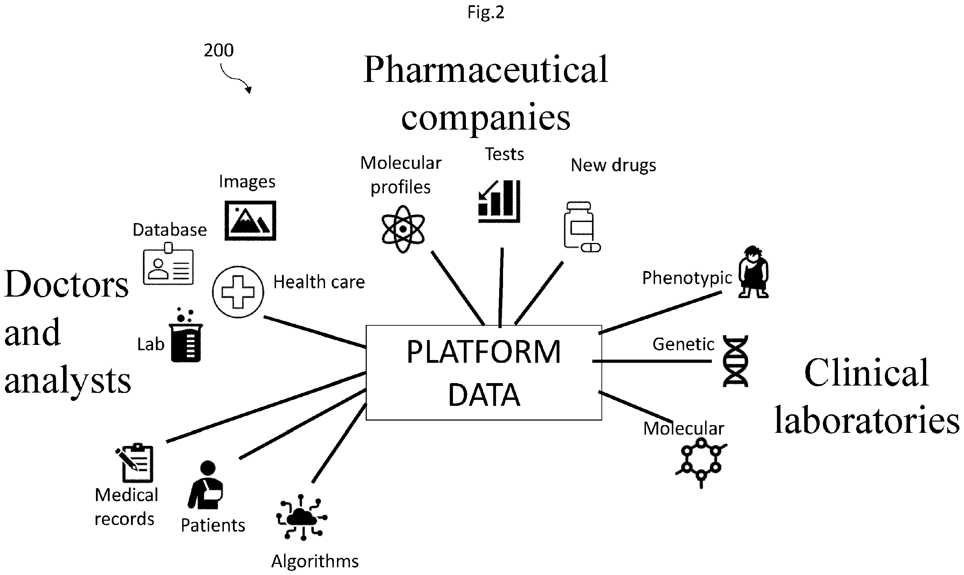

Fig.2 shows schematically the different categories of users to which the analytical platform is addressed and the different types of data that are exchanged with each category of users.
Fig.3 schematically illustrates the logic diagram (300) that has been realised for the development of the analytical platform object of the present invention.
Fig.4 shows the sequence diagram (400) illustrating the process of updating files according to an embodiment of the present invention.

### DETAILED DESCRIPTION

The present invention is the result of a long research path started in 2006, concerning the design of innovative techniques for the analysis of molecular networks. The research carried out in this area has led to the development of information and automated systems. The platform object of the present invention, called "PHOENIX eHealth Platform" and hereinafter referred to as PHOENIX, aims to transfer this experience in the industrial context of Precision Medicine, creating services that allow the end user to efficiently analyse huge amounts of data, storing them in a Cloud environment and easily displaying the results through a web interface.

In the description below, the term "Big Data" refers to the huge amount of data generated by the Internet and the digitisation of all types of information, including patient records, omics data and drug-target associations. "Real Time Analytics" refers to the analysis of Big Data, which includes the technologies and processes by which data must be measured, managed and analysed in real time as soon as it enters the system. The term Artificial Intelligence (AI) refers to the discipline that encompasses the theories and practical techniques for the development of algorithms that enable machines (particularly computers) to show intelligent activity, at least in specific domains and application areas.

The analytical platform, object of the present invention, provides the availability of software services in a "Cloud" environment to support doctors and healthcare professionals in the field of Precision Medicine. The software services provided by the platform are based on the combination of Artificial Intelligence, Big Data and Realtime Analytics within a plurality of data types, such as:
- omics data, namely data from the omics sciences, including genomics, proteomics, epigenomics, transcriptomics
- clinical data, e.g. from medical reports and patients' medical records;
- phenotypic data, namely the set of characteristics manifested by living organisms, such as morphology, development, biochemical and physiological properties including behaviour;
- "drug-target" associations, which take into account the molecular targets of certain drugs;
linking these data to the specific characteristics of the patients under investigation.

The PHOENIX platform integrates all the above types of data from a variety of different sources, such as: public and private databases; independent collection sources such as manually curated annotations by team members for gene and disease association; and annotations from other sources.

### Algorithms designed and implemented

The entire process flow is managed by combining specific algorithms. Since the architecture of the platform is implemented in the form of microservices, it is by its nature modular and extensible, so the number of algorithms and the algorithms themselves designed and implemented is constantly evolving. In particular, the algorithms that characterise the application "core" of the platform are original and specifically designed within the framework of the basic functionalities, by composing and modifying them others can be obtained.

The following is a summary of three families of algorithms designed and implemented within the PHOENIX platform that underpin some fundamental operations: (1) Integration - the integration of omics, clinical and phenotypic data, through the creation of complex functional networks; (2) Risk Factors - the determination of risk factors associated with the onset of certain diseases; (3) "Drug Effects" - the prediction of possible effects by a certain medicine on diseases other than those for which the medicine is intended.

### Integration

Data from a variety of heterogeneous sources, such as public databases, which include, for example, data produced in laboratories or patient registries, are found in various different formats, which are often incompatible with each other and therefore difficult to use for quick matching or comparison. The most commonly used formats include, for example, MI-TAB, Json, XML. For the purposes of data integration and modelling, a distinction must be made between two types of information, which can be derived from the available data:
- components;
- associations between the components.

Components, which are the "actors" in the system, comprise, among others, cellular components, e.g. genes, proteins, RNA and diseases. Associations are relationships between components that can be derived from laboratory experiments (e.g., protein-protein interactions), from literature (e.g., gene-disease association) or from other information directly provided as input to the platform and/or extracted from specific databases (e.g., patient registries).

A functional network is defined as an undirected graph FN(*N,A*) where N is the set of nodes, generally associated with the components, and A is the set of arcs, representing the associations between the components. Depending on the information present in the sources that feed the platform, each arc of the network can be associated with one or more scores, calculated by special algorithms designed and developed within PHOENIX.

The Integration algorithm receives a set of input files (data) and generates an output functional network (output), which integrates all the information:
**Input:** *n* files {*d1, d2,* ..., *dn}* from *n* different data sources;
**Output:** a functional network *FN* integrating all information;
*FN* = emptyset;
**for** *i* = 1, ..., *n*
*FN* = IntegrateSource (*FN, di*).

Each input file is "parserized" (parsing is a technique used in computing to convert data, e.g. an input file into a defined structure) and the information to be associated with the nodes and the information to be associated with the arcs of the graph corresponding to the existing (or to be created) network is specified. Initially, the functional network is empty. As the "parserization" of the files proceeds, a pair of nodes is extrapolated from the analysis of the data contained in the source and is added to the network as a new arc only if it is not already present; in both cases, the scores associated with the arc are calculated/updated, together with other auxiliary information (e.g., the list of "pubmed" codes of scientific publications in which evidence of that arc has been found, etc.). The output will be a functional network where each arc is unique and, at the same time, contains all the integrated information relating to the relationship between the two nodes involved in the arc. It should be noted that both the extraction of the pair of nodes from the information of the source and the calculation/updating of the scores may not be simple, but in many cases require a specialised analysis resulting from the peculiar combination of algorithms, technologies and use of hardware implemented in the PHOENIX platform.

### Risk factors

Stratification of risk factors for disease co-occurrence allows to provide a ranking of the probability that, starting from one disease, other diseases may occur, using disease-to-marker and marker-to-marker interactions as a linking bridge between diseases.
**Input:** a reference disease *dis;*
   a list of markers *M*=*{m1, m2,* ..., *mk} for dis;*
   a functional network *FN;*
**Output:** a set of *l* pairs <disease, risk> *MR*=*{*<*dis1, r1*>*, <dis2, r2>,* ..., *<disl, rl>}* representing the diseases for which there is a certain probability of co-occurrence with the reference disease *dis,* each with a risk value that allows to return the ranking;
*N*=ComputeNeighborhood(*M*, *FN*);
*list*_*diseases*=ExtractDiseases(*N*, *FN*);
MR=ComputeRisk(list *diseases, dis, FN);*

The ComputeNeighborhood procedure receives as input the list of markers associated with the reference disease and the functional network and, through an appropriately defined "neighbourhood" analysis with respect to the specific domain of interest, returns the nodes of the network intercepted within the neighbourhoods of each genetic marker of the reference disease (the genetic markers are identified as some of the nodes of the functional network).

The ExtractDiseases procedure analyses the sub-network induced by the nodes in N and checks whether genetic markers of other diseases are present within this sub-network. Let *list diseases* be the list of the diseases thus intercepted, through complex algorithms of analysis of the graphs in the distributed.

The ComputeRisk procedure considers each disease in *list diseases* and extracts the lists of associated markers from the database integrated in PHOENIX. Then, it builds a matrix that relates the markers of the reference disease with the markers of the other intercepted diseases and, through complex calculations, extrapolates the values associated with the risk factors of co-occurrence between the latter and the disease *dis.*

Even for the algorithms involved in determining risk factors, the interaction with technologies and hardware (Big Data, cloud, distributed computing) are fundamental to the implementation of the solution, and the corresponding peculiarities form an integral part of the invention itself.

### Drug Effects

The aim of these PHOENIX functions is to determine whether a certain medicine, which is used for a specific disease, can also have an indirect influence on other diseases.

Each drug affects a list of proteins (its targets). Each disease has associated markers (genes), which may have a link to the onset or course of that disease. Genes and proteins always have a causal relationship, since a specific gene produces a specific protein.

Given a drug and its targets, and given the relationships between targets and disease markers, the algorithms devised aim to establish which potential diseases this drug has a direct or indirect impact on.
**Input:** a drug *dg;*
   a list of targets *T*=*{t1, t2,* ..., *th}* for *dg;*
   a functional network *FN;*
**Output:** a set of *m* pairs <disease, impact> *MI*=*{*<*dis1, i1>, <dis2, i2>,* ..., *<dism, im>}* representing the diseases on which dg could have a certain influence, quantified by the value representing its impact;
*N*=ComputeNeighborhood*(T*, *FN);*
*list* diseases=ExtractDiseases(N, *FN);*
*MI*=ComputeImpact(*list_diseases, dg, FN);*

The procedure ComputeNeighborhood is analogous to the case of risk factors, with the difference that this time it will intercept the neighbourhoods of the nodes associated with the targets of *dg.*

The ExtractDiseases procedure analyses the sub-network induced by the nodes in N and checks whether genetic markers of other diseases are present within this sub-network. Let *list diseases* be the list of the diseases thus intercepted, through complex algorithms of analysis of the graphs in the distributed.

The ComputeImpact procedure considers each disease in *list diseases* and extracts the lists of associated markers from the PHOENIX integrated database. Then, through complex calculations, it returns the value of the impact that *dg* can have on each of the diseases under examination (after having carefully filtered out those markers that are directly targeted by *dg,* and the corresponding diseases). The considerations made above also apply to the algorithms involved here.

### Platform design.

In traditional Data Analytics applications, data is extracted from multiple sources by means of ETL (Extract/Transform/Load) processes, which perform Processing, Transformation and Loading operations in different "data warehouses". However, the heterogeneity of the data integrated in the analytical platform required a different type of development approach: the technological infrastructure was created through the "Domain Driven Design" approach, namely the system functionalities were separated into several application domains, which are interchangeable with each other. To do this, it was decided to use a microservice architecture, namely the subdivision of the application into a series of smaller, specialized parts, called microservices, each of which communicates with the others via a common interface, the API (Application Program Interface). A microservice architecture was chosen for the development of the platform because microservices guarantee:
- Faster "Time-to-market": namely they allow development cycles to be shortened, guaranteeing more agile "deployment" and updates.
- Higher scalability: namely they can be deployed across multiple servers and infrastructures, as required.
- Resilience: namely each service is independent and its failure does not affect other services in the infrastructure.
- Accessibility: namely they are more manageable by developers and easier to understand, update and improve.

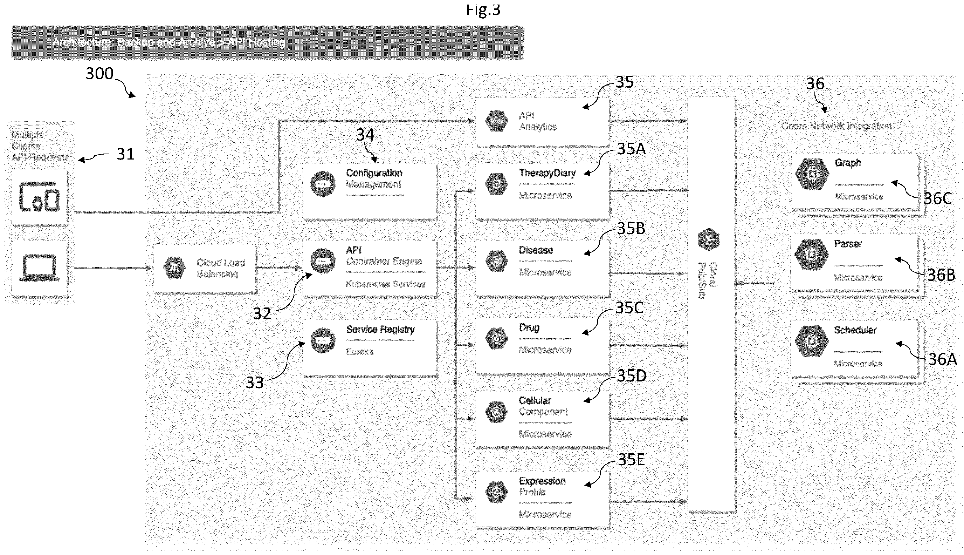

Fig. 3 shows the logic diagram (300) that was created for the development of the platform.

Users of the platform will be able to use Phoenix services through various tools, a web browser (e.g. Chrome, Edge, Firefox), mobile applications and using the services that the platform exposes. These tools are called clients and are referred to in Fig.3 as "Multiple Client API Request" (31).

A single exposed access point has been created, identified in Fig.3 as API Container Engine (32), which we will refer to later as API GATEWAY. This component has the task of providing an interface to the clients and is responsible for implementing the logic in a manner that is transparent to the "client".

This has a twofold advantage
- Any changes in the implementation of a service or the addition of a new one can also be made transparently to the "clients";
- there is only one level that needs to be protected externally from possible attacks. The paradigm shift has involved a radical change in communication mechanisms. In classical systems, communication was essentially 'synchronous', based on the use of the HTTP protocol (HyperText Transfer Protocol). In synchronous communication, the code "threads" remain blocked while waiting for a response from the server

Each PHOENIX microservice has a unique name that identifies it in the system and allows other services to correctly address requests. This name is stored in a registry, identified in Fig.3 as the "Service Registry" (33), which is queried by the system components to obtain the actual address. The process is similar to what happens with "Domain Name Systems". The service registry schema is an essential component for service identification. The registry consists of a database containing the network paths of the service instances. The service registry is constantly updated and highly available.

The PHOENIX architecture has been equipped with a distributed configuration system, identified in Fig.3 as "Configuration Management" (34), which allows each configuration of each microservice to be hot-loaded.

The PHOENIX system is equipped with an "analytics" mechanism, which makes it possible both to monitor the performance of the provided services and to provide external "analytics" to platform users, and which is identified in Fig.3 by the "API Analitics" component (35).

As seen above, the GATEWAY API (32) is the component which is delegated the task of addressing the requests received through the user interface, forwarding them to the appropriate services within the application. Communication from the user interface to the "gateway" takes place through the HTTP protocol, on a public network; subsequently, communication between the "gateway" and the internal services takes place on a private network, called "Phoenix Network".

Another function of the GATEWAY API (32) is to authenticate requests through a two-factor user authentication (2FA) mechanism, whereby the user is required to provide two different authentication factors to verify his identity. It relies on OAuth ("Open Authorization") as its authentication protocol, an open standard protocol that allows secure API authorization.

The "core" of the system, or "application core" is the component identified in Fig.3 as "Core Network Integration" (36), which implements the algorithms of the "Integration" group and therefore allows the integration of heterogeneous biological data from different sources, with the aim of linking various interactions and functional data, in order to allow the user to build specific functional networks for their own analysis.

Biological, clinical and pharmacological data are obtained by processing files extrapolated from heterogeneous sources, or provided directly as input by the user, or obtained through the analysis of literature, medical records, patient registries and other sources. Only in some cases, input files have a format that meets certain standards. For example, in the case of molecular interactions, one such standard is PSI-MI 2.5 and the corresponding format is MITAB25. This format describes binary interactions, one pair of interactors per row. The columns are separated by tabulations.

The file import and processing system is designed to be independent of the type of file to be processed. According to an embodiment, the "application core" takes the form of the development of three essential microservices:
- the "Scheduler Microservice" (36A), i.e. the application component that holds the burden of maintaining the control rules on the updating of the data deriving from the analysis of the input files;
- the "Parser Microservice" (36B), that is the component of the system that deals essentially with the processing phase of the input files;
- the "Graph Microservice" (36C), i.e. the component of the system that deals with the creation and maintenance of the specific functional network.

The input files are imported into the system through both automatic and manual procedures, including in the latter case a "form" for "uploading" the files into the system. The upload starts the file processing procedure. The automatic procedure is activated periodically, so that the core system, at set intervals, "downloads" the files to be processed, checks whether the file is different from the previously processed version, and if so, "downloads" the file and starts the processing procedure. The conclusion of the processing procedure is the generation or updating of the functional network.

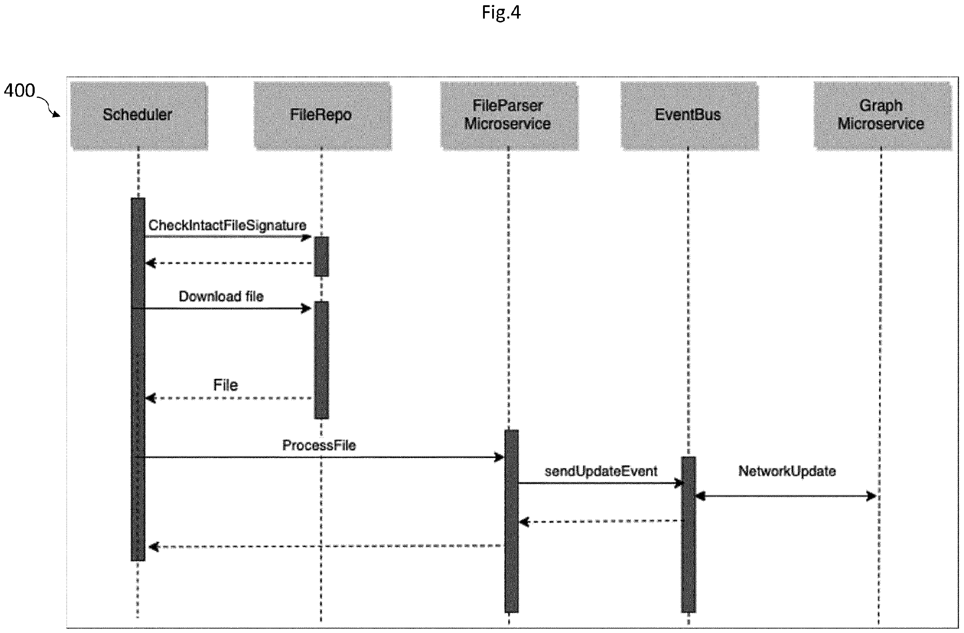

Fig.4 shows the sequence diagram (400) describing the "Automatic file import" process. According to one form of implementation, the microservice called 'Therapy Diary' (35A), has the task of processing and storing all phenotypic data from medical reports, linked to specific patient characteristics.

The system encrypts sensitive patient data using an encryption algorithm "SHA256" ("Secure Hash Algorithm") and a symmetric key, namely a keyword used to encrypt and decrypt the information, this key is stored in both the client and the server. Through this system a unique code is generated to identify the patient, with no possibility of linking the patient to their own sensitive information.

According to one embodiment, the microservice named "Disease" (35B) implements the algorithms of the group identified above as "Risk Factors" and has the task of processing and storing data on diseases, markers and disease-marker interactions. The main function of the microservice is to calculate risk factors for the co-occurrence of diseases and to provide a ranking of probabilities, so that it is possible to understand how, starting from one disease, others can occur.

According to one embodiment, the microservice named "Drug" (35C) implements the algorithms referred to above as "Drug Effects" and thus has the task of processing and storing data relating to medicines, with the aim of determining whether a certain medicine, which is taken for a specific disease, can also have an indirect influence on other diseases.

### Functioning of the platform

The functioning of the platform, according to a high-level description, can be summarized in four distinct phases:
1. integration of huge amounts of data from a plurality of heterogeneous sources (public databases, private databases, independent collection sources);
2. modelling of the integrated data through complex functional networks capable of providing a unified and functional view of all the integrated heterogeneous information;
3. data processing through efficient and sophisticated graph analysis algorithms for the extraction of hidden information;
4. production of suggestions and predictions in the field of Precision Medicine, provided on the basis of the newly generated knowledge.

Since the platform is organized according to a type of microservices architecture running in a cloud environment, and the data volumes and complexity involved are very high, the interaction with the hardware is important. In particular, the choice of the frameworks and the consequent design and development of the software are functional to the fact that the system works in a cloud environment, namely the provision of the services offered at the request of the end customer takes place through the Internet (e.g. through data storage, processing or transmission), starting from a set of pre-existing resources, configurable and available remotely in the form of a distributed architecture.

In order to manage such a large amount of data of different formats, traditional systems cannot be used, such as relational Database Management Systems (DBMS), where several interrelated data tables are used, running on a single machine. For data storage in PHOENIX, the choice was made to use NoSQL (Not only SQL) databases. These data stores, unlike those built on the basis of the relational model, do not assume a rigid structure, namely a schema, where the properties of the data and the relationships between them are described. NoSQL databases can be implemented following different approaches depending on the data structures with which the data records are represented. In particular, NoSQL databases can be divided into four main categories:
- **Key/Value:** data stored in an element that contains a key together with values (hash table).
- **Column Family:** data still organized as a "hash table", but using two or more levels of indexation.
- **Document store:** data stored within a document which may contain a structure with several fields and several lengths.
- **Graph based:** information stored through graph structures, making access more efficient with object-oriented languages and applications.

For the purposes of the platform object of the invention, the databases that are best suited to be used are those of the last two categories, namely "Document store" and "Graph based".

In the development of PHOENIX, a hybrid development approach was adopted, which makes use of both object-oriented and functional programming. Among the main paradigms adopted for the management of Big Data and the interaction with hardware in the distributed environment are MapReduce and Resilient Distributed Dataset (RDD). The first consists of a methodology of code drafting that allows the use of distributed hardware in an efficient manner and is partly transparent to the programmer. The latter allows distributed data storage to be optimized and made more efficient.

Thanks to the peculiarities of the algorithms, technologies and data involved, the PHOENIX platform is an effective automatic tool for extracting useful knowledge from the billions of accumulated data, allowing users to exploit hidden information in order to decide on the best course of action in the most complex cases, where their intuition, professionalism and experience are not sufficient due to the absence of explicit information.

There are in fact numerous diseases (e.g. multiple myeloma) for which standard protocols are only defined up to a certain stage (e.g. first relapse) or not at all, and the variety and phenotypic diversity of the patients involved is very high, so the doctor does not in fact have concrete tools to decide which of the various pharmacological options is the most appropriate for a given patient. Basically, in all these cases professionals are forced to proceed by trial and error, which very often considerably worsens the clinical condition of the patient, who runs the risk of being delayed or never receiving the most appropriate treatment for his case.

The data integrated in the platform are modelled through complex functional networks that allow the interrelation of extremely heterogeneous data that often do not show obvious correlations. On the basis of this data integration and modelling, PHOENIX performs complex analyses that allow the platform to provide various services for decision support, such as for example the calculation of risk factors associated with the onset of pathologies and possible "side effects" in the cases under examination, the suggestion of specific clinical analyses to be requested according to the situations, the discovery of new targets and/or the reallocation of drugs.

According to one embodiment of the invention, the data that can be processed by the platform relate to cellular interactions (e.g., protein-protein, mRNA-protein, etc.), biological functionalities (e.g., derived from biological ontologies), genotype-phenotype associations (e.g., genemalemia), sequencing (e.g., RNAseq).

According to one embodiment, the platform object of the present invention also enables processing of (public) stem cell data.

### Business model

The chosen business model is that of a multi-sided platform. The multi-sided platform involves three main categories of distinct, but interdependent users, to whom specific integrated and appropriately modelled data analysis services will be addressed. In particular, the platform provides:
- Services dedicated to doctors and analysts working within clinical institutions in the field of Precision Medicine, for the categorization of patients and automatic decision support for the formulation of specialized therapies.
- Services for clinical laboratories for the analysis of clinical results and the automatic suggestion of new tests to be performed, depending on the results obtained.
- Services dedicated to pharmaceutical companies that will allow analysts to study the associations between molecular profiles and sensitivity to specific active ingredients, providing automatic support for "drug design" and reduction of "side effects".

In particular, the platform provides a different user interface for each category of users:
- Phoenix Med (dedicated to doctors and MP analysts)
- Phoenix Lab (dedicated to clinical laboratories)
- Phoenix Pharm (dedicated to pharmaceutical companies).

Finally, it is clear that numerous modifications and variations can be made to the present invention, all of which fall within the scope of protection of the invention as defined in the appended claims.

## Claims

1. Analytical platform configured to provide a plurality of users with the availability of software services for data integration, modelling and analysis, said software services comprising one or more microservices, comprising
- a user interface (31) allowing at least a single user to enter requests
- an API (Application Program Interface) (32), which receives requests entered by at least one single user via the user interface (31), performs the authorization of said at least one single user, addresses the requests by initializing one or more microservices;
- a main module, or "Application Core" (36) for integrating a plurality of heterogeneous biological data from a plurality of sources, linking together the various interactions between said plurality of data, facilitating the creation by said at least one single user of specific functional networks for performing customized analyses.

2. Analytical platform according to claim 1 wherein said main module, or "Application Core" (36), comprises a first, a second and a third microservice, said first microservice comprising a "Scheduler" module (36A) for "downloading" data, said second microservice comprising a "Parser" module (36B) for processing data, said third microservice comprising a "Graph" module (36C) for creating the functional network.

3. Analytical platform according to claim 1 wherein said one or more microservices comprise:
- a microservice (35A) for processing and storing phenotypic data from medical reports;
- a microservice (35B) for processing and storing disease, marker and disease-marker interaction data;
- a microservice (35C) to process and store data on medicinal products, and to determine whether a medicinal product may also have an indirect influence on diseases other than the one for which it is used.

4. Analytical platform according to claim 1, wherein said plurality of heterogeneous biological data comprises information relating to molecular interactions, gene expression, transcriptomics, stem cells, epigenomics, clinical data and phenotypic data, "drug-target" associations.

5. Analytical platform according to claim 1, wherein said plurality of sources comprises public and private databases and independent collection sources.

6. Analytical platform according to claim 1, wherein said plurality of users comprises doctors and analysts working within clinical institutions in the field of Precision Medicine, clinical laboratories, pharmaceutical companies.

7. Analytical platform according to any one of the preceding claims, wherein said analytical platform operates in a "cloud" environment and is configurable as an application based on the internet network.

8. A method for providing availability of software services in a cloud environment for integration, modelling and analysis of data to a plurality of users, said method comprising:
- providing an analytical platform based on a microservice architecture for implementing the data integration, modelling and analysis steps, said steps comprising integrating a plurality of data types from a plurality of heterogeneous sources, modelling said plurality of data types by creating specific functional networks, processing said plurality of data types by graph analysis algorithms for extracting hidden information, generating hints and predictions based on the generated knowledge;
- providing a user interface (31) to allow at least a single user to enter requests;
- receiving and handling said requests from the user interface via an API Gateway interface program (32);
- directing said requests to one or more microservices via said API interface (32);
- integrating a main module, or "Application Core" (36) to connect said plurality of data types, facilitate the creation of specific functional networks, generate suggestions and predictions based on the generated knowledge;
- adopting models of said functional networks configured to correlate information, said information being uncorrelated if integrated directly from the sources without the adoption of said models, said information comprising a first and a second disease, said first disease having a first genetic marker and said second disease having a second genetic marker;
- providing resolution of problems related to Precision Medicine by implementing said models of said functional networks, said problems comprising the occurrence of said second disease as a "side effect" of said first disease as a result of drug therapy, and the choice of the best therapy for a specific patient.

9. Method according to claim 8, wherein said suggestions and predictions generated comprise calculating risk factors associated with the occurrence of diseases and possible "side effects", suggesting specific clinical tests to be required depending on the situation, discovering new "targets" or reallocating drugs.

10. Method according to claim 8, wherein said plurality of data types comprises molecular interactions, gene expression, transcriptomics, stem cells, epigenomics, clinical data and phenotypic data, and wherein said plurality of sources comprises public, private databases and independent collection sources.

11. Method according to claim 8, wherein said plurality of users comprises physicians and analysts working within clinical institutions in the field of Precision Medicine, clinical laboratories, pharmaceutical companies.
